# NEW EUROPEAN PATENT SPECIFICATION

(11) **EP 2 839 836 B2**
(45) Date of publication and mention of the opposition decision: **26.06.2024**
(45) Mention of the grant of the patent: 30.08.2017
(21) Application number: 14186663.2
(22) Date of filing: 14.09.2007
(51) Int. Cl.: A61K 35/74

(54) **Probiotics for use in reducing symptoms of respiratory disease**
Probiotika zur Verwendung bei der Reduzierung des Auftretens und der Dauer von Krankheiten
Probiotiques utilisés pour réduire l'incidence et la durée d'une maladie

(30) Priority: 02.10.2006 US 848662 P
(43) Date of publication of application: 25.02.2015
(62) Divisional of application: 07838266.0
(73) Proprietor: International N&H Denmark ApS, 2800 Kongens Lyngby (DK)
(72) Inventor: Leyer, Gregory, Palo Alto, CA 94304 (US); Ouwehand, Arthur, Palo Alto, CA 94304 (US)
(74) Representative: D Young & Co LLP

(56) References cited:
- WO-A1-98/23727
- WO-A1-2006/007526
- WO-A1-2006/054135
- US-A1- 2005 214 270
- US-A1- 2007 020 249
- RÍO MARÍA ESTHER ET AL: "[The nutritional status change the effectiveness of a dietary supplement of lactic bacteria on the emerging of respiratory tract diseases in children].", ARCHIVOS LATINOAMERICANOS DE NUTRICIÓN MAR 2002, vol. 52, no. 1, March 2002 (2002-03), pages 29-34, XP008173538, ISSN: 0004-0622
- DATABASE MEDLINE [Online] US NATIONAL LIBRARY OF MEDICINE (NLM), BETHESDA, MD, US; January 2000 (2000-01), MARUSHKO IU V: "[The development of a treatment method for streptococcal tonsillitis in children].", XP002732875, Database accession no. NLM10878987 & LIKARS'KA SPRAVA / MINISTERSTVO OKHORONY ZDOROV'IA UKRAÏNY 2000 JAN-FEB, no. 1, January 2000 (2000-01), pages 79-82, ISSN: 1019-5297
- OUWEHAND ARTHUR ET AL: "Probiotics reduce incidence and duration of respiratory tract infection symptoms in 3-to 5-year-old children", PEDIATRICS, vol. 121, no. Suppl. S, January 2008 (2008-01), page S115, XP002732874, & 25TH INTERNATIONAL CONGRESS OF PEDIATRICS; ATHENS, GREECE; AUGUST 25 -30, 2007
- SANDERS M E ET AL: "Invited Review: The Scientific Basis of Lactobacillus acidophilus NCFM Functionality as a Probiotic", JOURNAL OF DAIRY SCIENCE, AMERICAN DAIRY SCIENCE ASSOCIATION, US, vol. 84, no. 2, 1 February 2001 (2001-02-01), pages 319-331, XP026990170, ISSN: 0022-0302 [retrieved on 2001-02-01]

## Description

### FIELD OF THE INVENTION

The present invention provides a culture comprising *L. acidophilus* and *B. lactis* for use in reducing or preventing flu-like symptoms in a child affected by a respiratory tract infection, wherein *L. acidophilus* is strain NCFM and *B. lactis* strain is *B. lactis* Bi-07. In particular, the present invention provides a culture comprising *L. acidophilus* and *B. lactis* for use in reducing or preventing flu-like symptoms in a child affected by a respiratory tract infection, wherein *L*. *Acidophilus* is strain NCFM and *B. lactis* strain is *B. lactis* Bi-07 and wherein said child is between the ages of about 3 years and about 5 years.

### BACKGROUND OF THE INVENTION

Respiratory tract infections have been recognized as the most common of all infectious diseases. Upper respiratory tract infections include a large number of acute, inflammatory processes that primarily involve the nose, paranasal sinuses, middle ear, laryngeal-epiglottal tissues, and the oropharynx. These acute infections rank as the most frequent cause for patients of all ages to seek medical attention. In addition, a significant proportion of all infectious respiratory tract diseases are lower respiratory tract infections.

Although most upper respiratory diseases are self-limiting and benign, they represent a considerable socio-economic burden on populations. In contrast, lower respiratory tract infections often follow a life-threatening course that can seriously compromise the terminal alveolar pulmonary airway system, especially in individuals with altered pulmonary function.

Children and the elderly have a greater risk of severe disease due to upper and lower respiratory infections. Those in close proximity with other individuals have an even higher risk. Indeed, children attending day care centers have a 1.5 to 3 times increased risk of gastrointestinal and respiratory tract infections than children cared for at home or in small family groups (*See e.g.*, Hatakka et al., BMJ 322:1-5 [2001]). In addition to the direct medical costs associated with such infections, there are indirect costs incurred for parental leave from work to care for sick children. The lack of effective vaccines for many of these diseases contributes to the burden associated with these diseases. Thus, compositions and methods that help prevent and/or reduce the severity of respiratory diseases are needed. Río María Esther et al. (Archives Latinoamericanos de Nutrición, vol. 52, no. 1, March 2002 (2002-03), pages 29-34) reports the results of study into the effect of probiotics on respiratory tract infection in children. In contrast to the subject matter of the present claims, the probiotic combination consists of *L. acidophilus* and *L. casei.*

### SUMMARY OF THE INVENTION

The present invention provides a culture comprising *L. acidophilus* and *B. lactis* for use in reducing or preventing flu-like symptoms in a child affected by a respiratory tract infection, wherein *L. acidophilus* is strain NCFM and *B. lactis* strain is *B. lactis* Bi-07.

In some preferred embodiments, the child is between the ages of about 3 years and about 5 years. In yet additional embodiments, administering is conducted during the fall or winter months. In some embodiments, the culture is administered by mouth. In some preferred embodiments, the culture is provided in at least one nutritional supplement. In yet additional embodiments, the cultures reduce the need for antimicrobial administration to treat respiratory tract infection in a child. In still further preferred embodiments, the cultures prevent absenteeism due to said respiratory disease by a child.

The present invention further provides for the use of a culture comprising *L. acidophilus* and *B. lactis* in the manufacture of a medicament for reducing or preventing flu-like symptoms in a child affected by a respiratory tract infection, wherein *L. acidophilus* is strain NCFM and *B. lactis* strain is *B. lactis* Bi-07.In additional preferred embodiments, the child is of preschool age. In some preferred embodiments, the child is between the ages of about 3 years and about 5 years. However, it is not intended that the present invention be limited to this age range, as it is contemplated that the present invention will find use with various other ages. In yet additional embodiments, administering is conducted during the fall or winter months. However, it is not intended that the present invention be limited to these particular seasons, as it is contemplated that the present invention will also find use in other seasons. In some embodiments, the culture is administered by mouth. In some embodiments, the culture is provided in at least one nutritional supplement. In a yet additional embodiment, the use of the present cultures reduce the need for antimicrobial administration to treat respiratory tract infection in a child. In still further preferred embodiments, the use of the cultures according to the present invention prevent absenteeism due to said respiratory tract infection by a child.

### DESCRIPTION OF THE FIGURES

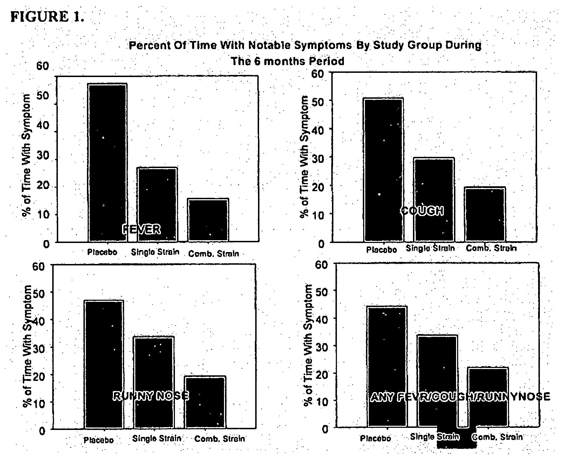

Figure 1 provides graphs showing the percent of time with notable symptoms by study group.

### DESCRIPTION OF THE INVENTION

The present invention provides a culture comprising *L. acidophilus* and *B. lactis* for use in reducing or preventing flu-like symptoms in a child affected by a respiratory tract infection, wherein *L. acidophilus* is strain NCFM and *B. lactis* strain is *B. lactis* Bi-07.

During the development of the present invention, it was found that significant protective effects in prevention flu-like symptoms (e.g., fever, cough, runny nose, etc.) were provided by *L. acidophilus* alone and in combination with *B. animalis* subsp. *lactis.* In addition to preventing flu-like symptoms, the administration of these organisms alone or in combination resulted in a significant reduction in the number of days individuals were symptomatic.

Thus, the present invention provides many benefits in terms of reducing or preventing flu-like symptoms in a child affected by a respiratory tract infection , which also reduces the economic impact of disease on families (e.g., direct medical costs), as well as parental absenteeism from work to care for sick children. Due to the significant impact respiratory infections represent, much work has been directed toward prevention and reduction of severity of such diseases. For example, nutritional supplements have been developed that claim to be effective in improving the health of children, including a probiotic combination comprising *B*. *lactis* (BB-12) and *L. reuteri* (ATCC 55730). Administration of formula supplemented with this probiotic to infants in child care centers was found to result in fewer and shorter episodes of diarrhea in the infants but did not impact the respiratory symptoms (Weitzman and Alsheikh, Pediatrics 115:5-9 [2005]).

During the development of the present invention, the effects associated with the consumption of probiotic products by preschool age children were assessed. These products were taken twice daily for six months during the time of year when illness is more prevalent in children, as well as adults (i.e., mid-September through mid-February). In particular, the consumption of probiotics in standardized dairy products (*e*.*g*., 1% fat milk or another probiotic product) was assessed for reduction in flu-like symptoms of illness in preschool age children attending day care centers. The number of days that a child was sick, but attended day care/pre-school, as well as the recovery period from the initial illness were also included in this assessment. In addition, the presence/absence of other diseases, as well as nutritional parameters (including body weight gain or loss) were also assessed.

### Definitions

Unless otherwise indicated, the practice of the present invention involves conventional techniques commonly used in food microbiology, nutritional supplements, pediatric disease, epidemiology, molecular biology, microbiology, protein purification, and industrial enzyme use and development, all of which are within the skill of the art.

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Margham, The Harper Collins Dictionary of Biology, Harper Perennial, NY (1991) provide those of skill in the art with general dictionaries of many of the terms used in the invention. Although any methods and materials similar or equivalent to those described herein find use in the practice of the present invention, preferred methods and materials are described herein. Accordingly, the terms defined immediately below are more fully described by reference to the Specification as a whole. Also, as used herein, the singular terms "a," "an," and "the" include the plural reference unless the context clearly indicates otherwise.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein, the term "food" refers to any nutritional item that provides nourishment to a plant and/or animal. It is not intended that the term be limited to any particular item, as it is used in reference to any substance taken into and assimilated by a plant or animal to keep it alive. It is also not intended that the term be limited to "solid" food, as liquid nourishment is encompassed by the definition. Indeed in some embodiments, liquid nourishment is preferred over solid food items. In some preferred embodiments, the term is specifically used in reference to food for human consumption.

As used herein, the term "feed" refers to any nutritional item that provides nourishment to non-human animals. It is not intended that the term be limited to any particular item, as it is used in reference to any substance taken into and assimilated by a plant or animal to keep it alive. It is also not intended that the term be limited to "solid" food, as liquid nourishment is encompassed by the definition. Indeed in some embodiments, liquid nourishment is preferred over solid food items.

As used herein, the terms "nutritional supplement" and "dietary supplement" refer to any product that is added to the diet. In some particularly preferred embodiments, nutritional supplements are taken by mouth and often contain one or more dietary ingredients, including but not limited to vitamins, minerals, herbs, amino acids, enzymes, and cultures of organisms.

As used herein, the term "neutraceutical" refers to a food/dietary supplement that is believed and/or taken to provide health benefits.

As used herein, the term "probiotic" refers to a live microbial food ingredient that is beneficial to health.

As used herein, the term "prebiotic" refers to a non-digestible food ingredient hat beneficially affects a human and/or other animal that ingests the prebiotic. In preferred embodiments, prebiotics selectively stimulate the growth and/or activity of at least one type of bacteria in the intestinal tract, such that the health of the human and/or other animal is improved.

As used herein, the term "synbiotic" refers to a mixture of prebiotics and probiotics.

As used herein, the terms "illness" and "disease" refer to any deviation from or interruption of the normal structure and/or function of any body part, organ, or system that is manifested by a characteristic set of symptoms and signs. The term encompasses conditions with known or unknown etiology and/or pathology.

As used herein, the term "treating" refers to the providing of compositions that result in the improvement, amelioration, and/or remedying of a disease, disorder, or symptom of disease or condition.

As used herein, the terms "oral administration," and "per os" refer to the taking of food and/or supplements by mouth.

As used herein, the terms "prevention of illness" and "prevention of disease" refer to measures taken to avoid the incidence of illness/disease. In some embodiments, "prophylactic" measures are taken in order to avoid disease/illness.

As used herein, the term "symptom of disease" refers to any subjective of disease and/or a patient's condition. It is used in reference to any such evidence as perceived by the patient.

As used herein, the term "sign of disease" refers to an indication of the existence of disease/illness. It is used in reference to any objective evidence of disease that is perceptible to the examining physician and/or other healthcare provider.

As used herein, the term "absenteeism" refers to the rate of absence from daycare and/or preschool due to illness. It also refers to absence from school and/or work due to illness. In some particularly preferred embodiments, the cultures of the present invention reduce the absenteeism of children from daycare and/or preschool due to illness (*e.g*., respiratory disease).

As used herein, the term "morbidity" refers to illness/disease.

As used herein, the term "mortality" refers to death.

As used herein, the term "incidence" refers to the rate at which a certain event occurs, as in the number of new cases of a specific disease that occur during a certain period of time.

As used herein, the term "prevalence" refers to the total number of cases of a specific disease and/or condition in existence in a given population at a certain time.

As used herein, the term "respiratory tract" refers to the system that is involved with breathing. The respiratory tract is often divided into three segments, namely the upper respiratory tract (*i.e*., nose, nasal passages, paranasal sinuses, throat/pharynx), the respiratory airways (*i*.*e*., larynx, trachea, bronchi, and bronchioles), and the lower respiratory tract (*i*.*e*, the lungs, comprised of respiratory bronchioles, alveolar ducts, alveolar sacs, and aleveoli).

As used herein, the term "respiratory disease" refers to any disease of the respiratory tract.

As used herein, the terms "flu" and "influenza" refer to contagious respiratory disease caused by any of the influenza viruses.

As used herein, the term "flu-like symptoms" refers to symptoms commonly associated with influenza, including but not limited to coughing, runny nose, nasal congestion (i.e., "stuffy nose"), sore throat, fever, muscle ache (*i.e.*, myalgia), stomach ache, headache, malaise, diarrhea, vomiting, ear ache, otitis media, etc.).

As used herein, the term "sequelae" refers to illness/disease and symptoms/signs that occur as a consequence of a condition and/or disease event. In some embodiments, sequelae occur long after the initial disease/illness has resolved.

As used herein, the term "sub-clinical infection" refers to infection that does not result in the production/observation of signs or symptoms of disease. Often, the patient is infected with a disease-causing organism, but is unaware of the infection.

As used herein, the term "infection" refers to the invasion and multiplication of pathogenic microorganisms in the body.

As used herein, the term "gastrointestinal tract" ("GI") refers to the entire alimentary canal, from the oral cavity to the rectum. The term encompasses the tube that extends from the mouth to the anus, in which the movement of muscles and release of hormones and enzymes digest food. The gastrointestinal tract starts with the mouth and proceeds to the esophagus, stomach, small intestine, large intestine, rectum and, finally, the anus.

As used herein, the term "gastrointestinal flora" refers to the microorganisms that inhabit the gastrointestinal system of humans and other animals. In some particularly preferred embodiments, the term is used in reference to bacterial organisms, but is not intended that the term be so limited.

As used herein, the terms "child" and "children" refers to young human beings under 18 years of age.

As used herein, the term "infant" refers to a child under one year of age. A "neonate" is a recently born infant (*i.e*., from birth to about four weeks of age).

As used herein, the term "toddlers" refers to children who are learning to walk. Generally, the term is used in reference to young children between one and three years of age.

As used herein, the term "preschoolers" refers to children who are attending pre-school, as well as children who are too young to attend kindergarten. In some preferred embodiments, the term is used in reference to children between the toddler and school-age groups (*e.g*., between approximately two years of age and five years of age).

As used herein, the term "school-age children" refers to children who are of a suitable age to attend school, in particular kindergarten through high school.

As used herein, the term "culture" refers to any sample or item that contains one or more microorganisms. "Pure cultures" are cultures in which the organisms present are only of one strain of a particular genus and species. This is in contrast to "mixed cultures," which are cultures in which more than one genus and/or species of microorganism are present. In some embodiments of the present invention, pure cultures find use. For example, in some particularly preferred embodiments, pure cultures of *Lactobacillus* (*e.g.*, *L. acidophilus*) find use. However, in alternative embodiments, mixed cultures find use. For example, in some particularly preferred embodiments, cultures comprised of *L. acidophilus* and *Bifidobacterium* find use.

As used herein, the term "*Lactobacillus*" refers to members of the genus *Lactobacillus*, in the family *Lactobacillaceae.* These bacteria are Gram-positive facultatively anaerobic bacteria that represent a major part of the bacterial group often referred to as "lactic acid bacteria." Various species of *Lactobacillus* have been identified, including but not limited to *L*. *acidophilus*, *L. bulgaricus*, *L. casei*, *L. delbrueckii*, *L. fermentum*, *L. plantarum*, *L. reuteri*, etc. *L. acidophilus* NCFM finds use in the present invention. It is intended that the genus include species that have been reclassified (*e.g*., due to changes in the speciation of organisms as the result of genetic and other investigations).

As used herein, the term "*Bifidobacterium*" refers to members of the genus *Bifidobacterium.* These bacteria are Gram-positive anaerobic bacteria that are one of the major strains of bacteria present in the gastrointestinal flora. *B. lactis* Bi-07 finds use in the present invention. It is intended that the genus include species that have been reclassified (*e.g*., due to changes in the speciation of organisms as the result of genetic and other investigations).

As used herein, the term "antimicrobial" refers to any compound which inhibits the growth or kills microorganisms. It is intended that the term be used in its broadest sense and includes, but is not limited to compounds such as antibiotics produced naturally or synthetically. It is also intended that the term encompass compounds and elements that are useful for inhibiting the growth of or killing microorganisms. In some preferred embodiments, the present invention reduces the need for administration of antimicrobials to children at risk or experiencing respiratory disease.

As used herein, the terms "microbiological media," "culture media," and "media" refer to any suitable substrate for the growth and reproduction of microorganisms. The term encompasses solid plated media, as well as semi-solid and liquid microbial growth systems.

As used herein, the term "fall months" refers to those months commonly recognized as occurring during the fall or autumn. In the northern hemisphere, these months include September, October and November. In the southern hemisphere, these months include March, April and May.

As used herein, the term "winter months" refers to those months commonly recognized as occurring during winter. In the northern hemisphere, these months include December, January and February. In the southern hemisphere, these months include June, July and August.

As used herein, the term "spring months" refers to those months commonly recognized as occurring during the spring. In the northern hemisphere, these months include March, April and May. In the southern hemisphere, these months include September, October and November.

As used herein, the term "summer months" refers to those months commonly recognized as occurring during the summer. In the northern hemisphere, these months include June, July and August. In the southern hemisphere, these months include December, January and February.

### EXPERIMENTAL

The following example is provided in order to demonstrate and further illustrate certain preferred embodiments and aspects of the present invention and are not to be construed as limiting the scope thereof.

In the experimental disclosure which follows, the following abbreviations apply: °C (degrees Centigrade); H₂O (water); gm (grams); µg and ug (micrograms); mg (milligrams); ng (nanograms); µl and ul (microliters); ml (milliliters); mm (millimeters); nm (nanometers); µm and um (micrometer); M (molar); mM (millimolar); µM and uM (micromolar); U (units); sec (seconds); min(s) (minute/minutes); hr(s) (hour/hours); sd and SD (standard deviation); PBS (phosphate buffered saline [150 mM NaCl, 10 mM sodium phosphate buffer, pH 7.2]); w/v (weight to volume); v/v (volume to volume); CFU (colony forming units); and NCFM (North Carolina Food Microbiology Department); Becton Dickinson (Becton Dickinson Diagnostic Systems, Sparks, MD); (Difco Laboratories, Detroit, MI); GIBCO BRL or Gibco BRL (Life Technologies, Inc., Gaithersburg, MD); ATCC (American Type Culture Collection, Manassas, VA).

The organisms used in the development of the present invention were *L. acidophilus* NCFM (PTA-4797) and *B. animalis* subsp. *lactis* Bi-07 (PTA-4802). These strains were grown at 37°C, in MRS medium (e.g., Difco, Becton Dickinson) containing 0.05% cysteine. Cultures were incubated for 48-72 hours in BBL GASPAK^{™} anaerobic jars containing H₂/CO₂ atmosphere (Becton-Dickinson). For large-scale production, the strains were grown in fermentation media, harvested by centrifugation and cryostabilized using methods known in the art. The cryostabilized solution was lyophilized. The lyophilized cultures were standardized to provide desired bacterial counts in each culture, using dextrose as a diluent for counting methods performed as known in the art.

The sachets used in the development of the present invention were produced by adding powder containing the probiotic material and a suitable carrier excipient to low moisture vapor transmission rate foil sachet packages to provide 5⁹ bacteria/g material. Two sachets were added to milk and consumed taken daily for a daily dose of 1¹⁰ bacteria. However, it is not intended that the present invention be limited to any particular dosage levels, as it is contemplated that a range of dosages will find use in the present invention.

In reviewing the data obtained during the development of the present invention, the information from the questionnaires, physician visits (when applicable), number of days of illness, incidences, duration, and episodes of specific illness (e.g., diarrhea) were compiled to determine what effects on health occurred from treatment with probiotics. Probiotic test Groups 1 and 2 received two doses of probiotics each day, seven (7) days per week, with administration occurring at the day care centers only for five (5) days per week and administration at home for the remaining 2 days per week. Group 3 (the placebo group), received a placebo twice daily seven (7) days per week, on the same schedule as Groups 1 and 2.

### A. Study Design

The study design consisted of a randomized, double blind, placebo-controlled study over a six month period. Subjects were assigned to the supplemented (Group 1 and 2) or control (Group 3) formula by block randomization procedure for each center and received the assigned formula for the duration of their stay in the center.

The participants in the study were pre-school age children between 3 and 5 years of age, non-gender specific, without pre-existing diseases or anatomic alterations conducive to frequent illness. Pre-school age children with contraindications to dairy products (e.g., lactose intolerance, or bovine protein reaction [cow's milk allergy]) were excluded from the study. In addition, participants who had history of inflammatory disease, intestinal disease, Crohn's disease, colitis, celiac disease, chronic cough from recurring respiratory distress-related diseases, Hirschsprung's disease, cystic fibrosis, or any other metabolic, neurological, anatomic alterations, such as symptoms of constipation or gastrointestinal functional distress (chronic diarrhea), were excluded from the study. In addition, children who were currently taking probiotic supplements in some other form (*e.g*., Yakult) were also excluded from participation. However, it is not intended that the present invention be limited to use for prevention of disease solely in children without pre-existing diseases and/or anatomic alterations conducive to frequent illness, as it is contemplated that the present invention will find widespread use and prove valuable in preventing disease in these groups.

As indicated above, there were three (3) groups, each of which contained 85 participants (*i.e.,* for a total of 255 children). Each of the three (3) groups was given an assigned alternate variable name rather than referencing them as "Group 1," "Group 2," and "Group 3." Thus, in some instances, the groups were referred to as 95, 94 and 93, as described below. Group 3 received the placebo 5 days per week, twice daily at the daycare center and 2 days per week, twice daily from home. Administration from home was provided by a parent, guardian, or designated family member. Administration from the daycare center was provided by a designated day care center representative. Groups 1 and 2 received probiotic products. Members of both Group 1 and Group 2 received the probiotic on the same administration schedule as Group 3. A typical daily dose for of the probiotic was 1¹⁰ to 10¹⁰ colony forming units/ml of strain. However, it is not intended that the present invention be limited to this specific dosage nor dosage regimen. In some preferred embodiments, the dosage is about 10⁸ to about 10¹² CFU/day, while in other preferred embodiments, the dosage is about 10⁹ to about 10¹¹ CFU/day. In addition, in some embodiments, the probiotic product is administered once per day, while in alternative embodiments, administration occurs twice or more each day. In some particularly preferred embodiments, administration occurs twice per day.

The 85 participants in Group 1 received a probiotic product containing *Lactobacillus acidophilus* NCFM (probiotic product A) and the 85 participants in Group 2 received a probiotic product containing two strains, namely *Lactobacillus acidophilus* NCFM and *Bifidobacterium*, Bi-07 (probiotic product B), with each strain comprising half of the daily dose.

The dosage for intake of the probiotic product (or sachet) for both Group 1 and Group 2 consisted of 170-250 ml of standard 1% fat milk. The probiotic products were contained in foil sachets for ease of use and transport. The dosages were administered as 5⁹ bacteria in 170-250 mls milk for a daily total dose of 10¹⁰ bacteria. The contents of the foil sachet were dispensed into the milk at the point of consumption. Foil sachets were provided that contained both placebos and probiotics, so that adding of the sachet contents to the milk was common to all of the Groups. Doses were provided twice daily for a total of 170-250 ml per day, for 6 months. These doses were taken with food, which consisted of a snack or a meal. The supplement was prepared in a preparation room with a temperature kept at 18 to 20°C. The supplement powder was added to the milk, stirred, and served to children within 5 minutes. Children received the supplement in a common designated area.

The study began in November with N=326 children (Group 3:104, Group 1:112 and Group 2:110) and ended in May with N=248 (Group 3:92, Group 1:77, and Group 2:79). The net difference between November and May was a loss of 78 children (Group 3:12, Group 1:35, and Group 2:31). There were 144 male and 182 female participants, ages 3 - 5 years, and all of Chinese ethnic origin.

The children arrived daily within the timeframe 6:40 a.m.- 8:40 a.m. and departed within the timeframe of 3:50 p.m. - 4:40 p.m. The only beverage available (outside of the study supplements) was water. The children's families consisted of farmers, government employees, and teachers. Children were required to obtain standard vaccines provided to children in China.

Based on the input of the local physician researcher as to the illnesses that are prevalent in local preschool children, this study allowed for the inclusion of the frequency of these illnesses to determine whether inclusion or consumption of either of the probiotic products (probiotic product A or probiotic product B) by these children led to a decrease of incidences of these illnesses. Indeed, based on 170 participants (Groups 1 and 2 combined), a t-test for detecting medium-to large effects was provided with at least a power of 80% and an alpha level of 0.05. The study also allowed for the inclusion of other parameters to be defined up-front by the local pediatrician/physician to determine the benefit of this product on the health of preschool children. The study was also used to determine whether antimicrobials could be used less often when the probiotic products are administered, thereby resulting in the decreased use of antimicrobials.

A questionnaire was provided for each participant, in order to determine qualifying and disqualifying factors (or inclusion and exclusion criteria) for participants. For example, participants' families were required to have adequate refrigeration for the product and the parent or guardian of each child (in each Group) was require to sign a waiver agreeing to provide the supplement at home and track and report any signs/symptoms of illness to the day care center representative.

The initial questionnaire was completed by the designated day center representative at each day care center site, utilizing information supplied by the parent or guardian. The parent and designated day care center representative provided initial verification of the information. The clinical coordinator (*i.e.,* a physician researcher) for the study collected the completed forms from all of the day care centers from the each of the day care center heads (*i.e.,* the persons in charge of the day care centers, or the principals). The day care center representative and the parent/guardian of each preschool child provided daily records on the symptoms check list form any symptoms of illness, absences and reason for absence from the day care center, any doctors' diagnoses, prescription (detail) of antimicrobials, and specific symptoms leading to absence (as not all symptoms result in an absence). The day care representative ensured coordination of information from the parent/guardian each time a child was absent from the center. The designated day care representative reviewed the symptom sheets daily and provided the symptom sheets to the clinical coordinator (physician) at regular weekly meetings that additionally included the head of the day care center. The designated daycare center representative, the head of the daycare center, (and the clinical coordinator (physician)) were all unaware of which formula was being fed to the control group and which formulae were being fed to the probiotic groups.

### B. Validation of Daycare Center Sites

Twelve to fifteen centers were examined by the physician researcher (or the physician's appointed nurse) with a final selection of eight (8) daycare centers selected in a westernized region of Asia. All of the probiotic products and all of the placebo products were kept in a cool storage area in a designated location in Asia for storage until delivery to the sites.

The daycare center sites were validated to ensure that study settings were comparable to each other. Therefore, centers were located that provide, in general, similar numbers of enrolled preschoolers, available staff, pricing for the daycare center services, and refrigeration facilities at each center. Questionnaires were used to gather information regarding the facilities, including questions regarding number of preschoolers, number of employees, classification of clientele (income), cost per preschooler, whether or not the preschoolers were fed by the center or brought their own food, the usual time the preschoolers were dropped off and picked up, etc. Completed questionnaires were returned through the postal service, by the day care center heads. Prior to selection, each center was evaluated for cleanliness and hygiene by the physician researcher or the physician researcher's appointed nurse. Prior to the start of the study, the physician researcher met with the owners of the daycare centers and staff to validate whether or not the questions were significant and to elicit feedback and evaluate tracking methods and tools, such as follow-up contact with parents regarding absenteeism. To further ensure that the questionnaires provided the appropriate data, the physician researcher ensured that the statistician performed the statistical analysis throughout the study, so that any problems with the study design could be identified and rectified to meet the needs of the study.

### C. Documentation of Attendance Forms

Each day, the day care center representative completed an attendance sheet. Each sheet listed the location, person in charge (*i.e*., who collected the information), the size of the class or group, and the participant's name. The check off segment asked if the participant was present (yes/no), whether or not the participant appeared sick (yes/no), whether the participant was exhibiting flu-like symptoms or other illness-related symptoms (yes/no), whether the participant had a diarrheal episode (yes/no). If there was a diarrheal episode, there was a question as to whether the participant had more than 2 diarrheal episodes in one day (yes/no), whether or not the first dose of product was taken (yes/no), and whether or not the second dose of product was taken (yes/no). If a child was absent, upon their return, sections regarding whether the child went to the doctor (yes/no), received any antimicrobials (yes/no) and whether the child received any other disease-related treatment (yes/no) were filled out. In order to validate data collection, random visits by the physician (or the physician's appointed nurse) were made to the day care centers, in order to review records and ensure daily completion of the records. The head of each day care center ensured that the day care center representative collected all of the questionnaires and any other related study forms for the physician researcher, and the head of the day care center ensured that this information was provided to the physician researcher. The participants' parents used a check list of symptoms to document symptoms of illness and whether or not the illness related to absenteeism.

Absenteeism was considered to be a measurement factor correlated with symptoms. Therefore, the effect of absenteeism on the symptoms encountered by children was evaluated. The absenteeism data were collected in a systematic and comprehensive fashion, without relying on parental or instructor recalling abilities, but rather official school records information only. Actual dates of absence were tracked for each of the flu-like symptoms, other illnesses, and personal or unknown reasons.

### D. Outcome Measures

Measurements included the number of days with illness-related or flu-like symptoms, the number of absences from day care because of illness or fever, the "flu" as diagnosed by a doctor, illness or "flu-like" symptoms and any pre-disposing factors, such as a course of prescribed antimicrobials. Other symptoms recorded included frequency and duration of diarrhea and any other illness-related symptoms. For example, other symptoms such as ear infections or headaches or reasons resulting in the participants' absence were noted. A general wellness score was determined for each participant. Separation of data of participants taking antimicrobials and probiotics allowed the separate tracking of these participants from those participants who only took probiotics, in order to ensure that the results were not skewed due to the administration of antimicrobials. In addition, the administration of antimicrobials to participants in the control group due to "flu-like" symptoms, was documented, as the antimicrobials may have decreased the number of days absent due to illness. In addition, as indicated above, the participants' weight were measured prior to the study and upon completion of the study.

During the course of the study, eight (8) symptoms were evaluated determined to be "flu-like" symptoms as per the symptoms table below, which included nausea, vomiting/diarrhea, body ache, headache, larynx ache (sore throat), runny nose, headache, and fever. Vomiting and diarrhea were treated as one category but separate recording of vomiting was tracked, and only six (6) children throughout the course of the study experienced vomiting. No secondary symptoms were noted during the course of the study.

The children visiting the hospital (the usual site for a doctor's visit), typically received treatment as per Western medicine protocols, in addition to traditional medicine. These visits typically included recording the child's temperature and prescribing antimicrobials as appropriate. It was noted that antimicrobial use was found to be more prevalent in the placebo group.

The relationship between the probiotic amount consumed and the number of days with symptoms was listed on the symptoms score sheet. Additionally, the number of days of absences due to fever, and symptoms of cold, number of diarrheal episodes (incidence and duration), and documentation of antimicrobial prescriptions (type of medication and dosage amount) were noted, as well as intake of other medications that are being taken due to illness-related symptoms. The log sheet of the amount of probiotic taken was recorded daily, and an intake of less than 200 ml per day flagged for the clinical coordinator. The symptoms score sheet was used to determine the degree of illness symptoms following each secondary measure.

### E. Statistical Analyses

The analyses plan included determining the balance among the three study groups with respect to age, gender, and weight; calculation of symptom rates by influential factors including age and study group; and univariate and multivariate determination of and testing for the magnitude and significance of differences among the groups in terms of symptoms rates and duration. Univariate testing of a significant difference among the groups for continuous variables like age and weight (to test their balance), employed analyses of variance (ANOVA) techniques. For absenteeism, summary statistics were provided for the three study groups, and included means, standard deviations, 25^{th}, 50^{th} (median), and 75^{th} percentile cut-points, as well as maximum and minimum values (*i.e*., outliers). Initial analyses of the absenteeism difference among study groups were investigated using ANOVA techniques. Comparisons of single and double strain groups versus placebo were carried out using Dunnett's tests for multiple comparisons.

Descriptive statistics were used for demographic variables (*e.g*., age, gender, and initial body weight), living environment, probiotic consumption amount, frequency of symptoms, etc. The numbers of days with illness, "flu-like" symptoms, other illness-related symptoms, or total absences due to illness were measured continuously by t-test, to allow multiple comparisons of different groups. These analyses were made following completion of the ANOVA test. Cox's or a similar regression analyses method which was performed to adjust for confounding factors (such as age). The number of "flu-like" symptoms and probiotic intake were dichotomized (none/one or more) and analyzed with the Fisher's exact or similar test. Partial correlations were calculated between milk (or designated product) consumption and the number of days with illness. ANOVA.

Multiple comparisons of the different groups were made using the t-test for primary outcome variables. Once the data and statistical differences were determined, then the t-test was used to determine which groups differed and the alpha was adjusted to avoid type I error inflation. A type I error was not expected to occur as there were 3 groups, with testing of the groups occurring over a period of time (6 months). At the end of the study, the chi-square test of independence was used when there was an end point, to see the results for comparing treated and non-treated participants based on the number of participants who did and who did not experience flu-like or illness-related symptoms. For these analyses, a total of 105 total participants were needed to secure a power of 90% and an alpha level of .05. However, it is contemplated that in other embodiments, a different power and/or alpha level (*e.g*., 0.05 alpha level) will find use. Statistical analyses were performed with SPSS or other computerized statistical package.

Testing among the groups for dichotomous variables, including the outcome measure (presence of symptoms) and gender, was performed using chi-square and/or Fisher exact methods. For the sake of adjusting for influential factors (*e.g*., "confounders") on the outcome measure, such as age and weight, logistic regression analyses were used to model the log of the odds of presence of a symptom as a linear function of age, study group weight and gender. The odds of having a symptom was equal to the probability of contracting the symptom divided over the probability of not contracting the symptom.

For example if 10 out of 100 individuals developed fever, then the probability of the symptom is 0.10 and the odds for the symptom are 1/9 (0.10/0.90). Presentation of the multivariate analyses uses the concept of the odds ratio. For example, the risk of contracting a specific symptom for children assigned to one group (*e.g*., the combination strains) relative to children assigned to another group (*e.g*., the placebo) was assessed. If the odds ratio is unity (1.0) or closer to 1.0, then the two groups analyzed are likely similar with respect to the risk for the symptoms. If the odds ratio is greater than one then there is a potential increase in risk, whereas if the risk is less than one then there is a potential decrease in risk. Results are presented using estimates for the odds ratio with 95% confidence limits. For example if the odds ratio of fever for the combination strain group relative to the placebo group is 0.33 and the corresponding confidence limit does not include the value "1" ; *e.g*., (0.15, 0.70) then it follows that the risk of fever for the combination strain group is about 1/3 of that of the placebo group. In other words the risk for fever has been decreased by 67% due to the affiliation with the combination strain group.

Interaction of age with study group was also investigated in the realm of the logistic regression modeling. As the analysis indicated, age was a possible interacting factor, even though at times its ability to act as a interaction factor did not reach statistical significance due to thinning of sample size when subgroups of strains (and placebo) by age were examined with respect to presence of symptoms. Therefore, results are presented overall (disregarding age as an interaction factor), although the logistic regression model results are presented by age, in recognition of its effect as an interacting factor. In addition, pair-wise group comparisons were further subjected to multiple comparison rules and penalties. In sum, evaluation of differences among the study groups with respect to symptoms duration was conducted using multiple linear regression analyses to model duration as a function of study group, age, gender and weight.

### F. Results

Table 1 provides the distribution of age, weight and gender among the three study groups. It is evident that the groups are balanced with respect to gender and to some degree, weight. However, it seems that children assigned to the placebo group were on average older than those assigned to single or combination strains groups by about 4-5 months. This finding was considered to necessitate inclusion (adjustment) for age on all subsequent analyses.

| **Table 1. Baseline Characteristics by Study Group** | | | | |
|---|---|---|---|---|
| | **Study Group** | | | |
| | **Placebo** | ***L*. *acidophilus* NCFM** | ***L. acidophilus* NCFM with B. *lactis* Bi-07** | **P-value** |
| | **(Group 3)** | **(Group 1)** | **(Group 2)** | |
| **AGE (yrs)** | | | | |
| N | 104 | 110 | 112 | |
| Mean + SD | 4.1 + 0.54 | 3.7 + 0.7 | 3.8 + 0.6 | <0.001 |
| 25^{th} Percentile | 3.9 | 3.1 | 3.3 | |
| Median | 4.2 | 3.5 | 4.1 | |
| 75^{th} Percentile | 4.5 | 4.3 | 4.1 | |

| **WEIGHT (lbs)** | | | | |
|---|---|---|---|---|
| **Table 1. Baseline Characteristics by Study Group** | | | | |
| | **Study Group** | | | |
| | **Placebo** | ***L. acidophilus* NCFM** | ***L. acidophilus* NCFM with *B*. *lactis* Bi-07** | **P-value** |
| | **(Group 3)** | **(Group 1)** | **(Group 2)** | |
| N | 104 | 110 | 112 | |
| Mean + SD | 17.1 + 2.30 | 18.0 + 5.4 | 16.9 + 2.0 | 0.06 |
| 25^{th} Percentile | 15.3 | 15.5 | 15 | |
| Median | 17 | 17 | 17 | |
| 75^{th} Percentile | 18.5 | 18 | 18 | |

| **GENDER** | | | | |
|---|---|---|---|---|
| N | 104 | 110 | 112 | |
| Males (%) | 44(42.3) | 47(42.0) | 53(48.2) | 0.58 |

Although symptom rates were evaluated by gender and weight, neither were found to have an appreciable effect on the probability of contracting symptoms (fever, cough, runny nose or any symptom). However, age seemed to have a significant effect (independent of study group) on the probability of the presence of symptoms as well as duration (p-values for all months were consistently < 0.05).

Statistically significant protective effects were observed for the strain groups in regards to the occurrence of fever, cough and runny nose. Table 2 provides data showing the distribution of symptoms duration over the six month follow up study period, by randomized groups. It also evaluates age adjusted differences among study groups. Based on this Table, it is clear that the placebo group experienced a longer duration of symptoms, by an average of 3.2 and 2.2 days relative to combination strains group (p<0.001) and single strain group (p=0.0023), respectively. For convenience in presenting these data, in this Table, the placebo group (Group 3) is denoted by the number "93", single strain group (Group 1) by the number "94" and combination strains group (Group 2) by the number "95."

| **Table 2. Distribution of Symptoms Duration (Days) by Study group Over the Six Month Follow Up Period** | | | |
|---|---|---|---|
| **Statistic** | **Placebo (93)** | ***L. acidophilus* NCFM (94)** | ***L. acidophilus* NCFM with *B. lactis* Bi-07 (95)** |
| N | 104 | 110 | 112 |
| Mean + Sd | 6.5 + 7.3 | 4.5 + 4.7 | 3.4 + 3.7 |
| 25^{th} Percentile | 1 | 1 | 1 |
| 50^{th} Percentile | 4 | 3 | 2 |
| 75^{th} Percentile | 10 | 6 | 5 |

| | **Age-Adjusted Differences Among the Groups** | | |
|---|---|---|---|
| | 95 vs. 93 | 94 vs. 93 | 95 vs. 94 |
| Mean + Sd | -3.2 + 0.76 | -2.17 + 0.71 | -1.06 + 0.81 |
| P-value | < 0.001 | 0.0023 | 0.195 |

For the age-adjusted differences among the groups, the cumulative duration of symptoms was modeled using linear regression as a function of study group and age. These differences reflect the regression coefficients in such models when accounting for the effects of age.

The results also indicated that during the six-month study period, the single (Group 1) and combination strains (Group 2) groups showed a significant decrease in the percentage of time that participants experienced the flu-like symptoms of fever, cough or runny nose. The following table (Table 3) provides data comparing the symptoms observed between the groups, as well as the administration of antimicrobials. In this Table, the odds ratios, 95% confidence level and P-values are provided. In this Table, an odds ratio less than 1 indicates a protective effect. For example, an odds ration of 0.52 in comparing Group 94 and Group 93 indicates that the children in Group 94 have about half the risk of developing the symptom as compared to those in Group 93. In this Table, the bold values indicate particularly significant results.

| **Table 3. Six Month Comparison of the Incidence of Symptoms and Antimicrobial Administration (Odds Ratios, Confidence Limits and P-Values)** | | | | | |
|---|---|---|---|---|---|
| **Group Comparisons** | **Symptom** | | | | |
| | **Fever** | **Cough** | **Runny Nose** | **Any Symptom** | **Antimicrobial Administered** |
| 94 vs. 93 | 0.52 (0.31, 0.88) | 0.58 (0.36, 0.94) | 0.91 (0.56, 1.46) | 1.06 (0.54, 2.1) | 0.32 (0.18, 0.59) |
| | P=0.013 | P=0.027 | P=0.68 | P=0.86 | P=0.0002 |
| 95 vs. 93 | 0.37 (0.20, 0.67) | 0.46 (0.27, 0.79) | 0.56 (0.33, 0.94) | 0.52 (0.27, 0.99) | 0.20 (0.09, 0.43) |
| | P=0.01 | P=0.005 | P=0.03 | P=0.045 | P<0.0001 |
| 95 vs. 94 | 0.71 (0.37, 1.36) | 0.80 (0.46, 1.40) | 0.62 (0.36, 1.10) | 0.49 (0.24, 0.98) | 0.63 (0.27, 1.47) |
| | P=0.30 | P=0.43 | P=0.088 | P=0.04 | P=0.29 |

Of 84 participants who were administrated antimicrobials, the majority belonged in the placebo group (Group 3), with 67.9%. In contrast, for the single strain group the percentage was 21.4%, and 10.7% for the combination strains group.

Except for the beginning of the study and possibly the month of March, both antimicrobial use and physician visits were more prevalent among the placebo group. One possible interpretation to such trend could be as a consequence of having more prevalence of flu-like symptoms (or other symptoms) that necessitated a physician visit. Also, diarrhea cases were relatively more prevalent at the beginning of the study, with no clear association to study group.

As indicated in Table 1, the placebo group had a significantly higher percentage (%) of older children relative to the two other strain groups throughout the months of the study. It was therefore somewhat expected that the placebo group would show more resistance to the flu-like symptoms. However, the older children showed more susceptibility to symptoms during the months of November-January, relative to younger children. Additionally, such an effect was either reversed or considerably diluted through the remainder months of February -May.

One possible explanation of this trend is that all of the children (younger or older) were highly vulnerable to flu-like symptoms during the coldest months (November-January) of the winter season. However, during these months, the strains effects were significantly protective against such symptoms. It is also interesting to note that following this conjuncture, the lack of balance of age among the study groups may have negatively impacted the potential benefit of the strains.

In sum, the results indicated that there were significant protective effects of the strain groups versus placebo in preventing flu-like symptoms of fever, cough and runny nose during the months of November, December, January, February, March, April, and May (any symptoms). There was a decline in protective effects observed during the months of April-May. However, there was some protection provided during these months. Indeed, overall, significantly fewer children suffered flu-like symptoms of fever, cough or runny nose among single or combination strains groups compared to children assigned to the placebo group. In addition, three times as many children in the placebo group as compared to the single strain group were prescribed antimicrobials due to flu-like symptoms. Moreover, five times as many children in the placebo group as compared to the combination strains group were prescribed antimicrobials due to flu-like symptoms. Furthermore, there were significantly fewer days that participants in the single and combination strains groups exhibited symptoms, as compared to the placebo group. The combination of *L. acidophilus* NCFM with *B. lactis* Bi-07 provided some superiority over the single strain (*L. acidophilus* NCFM) in preventing symptoms, although benefit was also observed for the single strain.

In addition, the present invention was found to provide benefits in both the single strain and double-strain groups, by reducing the number of sick days by 50%. It is evident from Table 4, that children assigned to the placebo group have had, on average, 37% - 44% more total absent days than those assigned to the single or double strain groups.

| **Table 4. Distribution of Total Absent Days by Study Group Over the Six Months Follow Up** | | | |
|---|---|---|---|
| **Statistic** | **Placebo (Group 93)** | ***L. acidophilus* NCFM (Group 94)** | ***L. acidophilus* NCFM with *B. lactis* Bi-07 (Group 95)** |
| N | 104 | 110 | 112 |
| Mean ± Std. Dev. | 5.2 ± 5.7 | 3.6 ± 3.7 | 3.8 ± 3.9 |
| Minimum | 0 | 0 | 0 |
| 25^{th} Percentile | 1 | 1 | 1 |
| 50^{th} Percentile | 3 | 3 | 3 |
| 75^{th} Percentile | 8 | 5 | 5 |
| Maximum | 26 | 18 | 23 |

Indeed, the data indicated that both the single and double strain groups demonstrate a significant reduction in number of absent days relative to the placebo group.

Table 5 presents results of statistical significance testing of the differences among the groups in total absent days using ANOVA techniques adjusting for multiple comparisons using Dunnett method. The difference between either the single strain or double strain group versus the placebo is statistically significant (*p*-value = 0.01). Additionally, as it is indicated by the simultaneous 95% confidence intervals that have been constructed adjusting for multiple comparisons, the difference comparing single or double strain group to the placebo is statistically significant. This difference could be as small as 0.1 or 0.24 days but could also be as high as 2.7 or 3.0 days in number of total absent days.

| **Table 5. Multiple Comparison Adjusted* Differences Among the Three Groups** | | | |
|---|---|---|---|
| | **94 v. 93**** | **95 vs. 93**** | **94 vs. 95** |
| **Mean** | -1.6 | -1.5 | -0.21 |
| **Simultaneous 95% Confidence Interval** | -2.9678 | -2.7389 | -1.1502 |
| | v. | v. | v. |
| | -0.2489 | -0.0569 | 1.5711 |
| * Using Dunnett for the multiple comparison adjustment | | | |
| **Statistically significant using overall 0.05 α-level. | | | |

In sum, analyses of total absent days indicate that single or double strain probiotics significantly decreases the number of absent days from kindergarten or preschool.

## Claims

1. A culture comprising *L. acidophilus* and *B. lactis* for use in reducing or preventing flu-like symptoms in a child affected by a respiratory tract infection, wherein *L. acidophilus* is strain NCFM and *B*. *lactis* strain is *B. lactis* Bi-07.

2. The culture of claim 1, wherein said child is between the ages of about 3 years and about 5 years.

3. The culture of anyone of claims 1 to 2, wherein said culture is for administration during the fall or winter months.

4. The culture of anyone of claims 1 to 3, wherein said culture is for administration to said child by mouth.

5. The culture of anyone of claims 1 to 4, wherein said culture is provided in a nutritional supplement.

6. The culture of anyone of claims 1 to 5, wherein said culture reduces the need for antimicrobial administration to treat said respiratory disease in said child.

7. The culture of anyone of claims 1 to 6, wherein said culture prevents absenteeism due to said respiratory disease by said child.

8. Use of a culture comprising *L. acidophilus* and *B. lactis* in the manufacture of a medicament for reducing or preventing flu-like symptoms in a child affected by a respiratory tract infection, wherein *L. acidophilus* is strain NCFM and *B*. *lactis* strain is *B*. *lactis* Bi-07.

9. The use of claim 8, wherein said culture is administered to said child by mouth, preferably as a nutritional supplement.

10. The use of anyone of claims 8 to 9, wherein said culture reduces the need for antimicrobial administration to treat said respiratory disease in said child.

11. The use of anyone of claims 8 to 10, wherein administering said culture reduces absenteeism due to said respiratory disease by said child.

## Patentansprüche

1. Kultur, umfassend *L. acidophilus* und *B. lactis,* zur Verwendung beim Verringern oder Vorbeugen grippeähnlicher Symptome bei einem von einer Atemwegsinfektion betroffenen Kind, wobei es sich bei dem *L. acidophilus-Stamm* um NCFM handelt und bei dem *B.* lactis-Stamm um *B. lactis* Bi-07 handelt.

2. Kultur nach Anspruch 1, wobei das Alter des Kinds zwischen etwa 3 Jahren und etwa 5 Jahren liegt.

3. Kultur nach einem der Ansprüche 1 bis 2, wobei die Kultur für eine Verabreichung während der Herbst- oder Wintermonate vorgesehen ist.

4. Kultur nach einem der Ansprüche 1 bis 3, wobei die Kultur für eine orale Verabreichung an das Kind vorgesehen ist.

5. Kultur nach einem der Ansprüche 1 bis 4, wobei die Kultur in einem Nahrungsergänzungsmittel bereitgestellt wird.

6. Kultur nach einem der Ansprüche 1 bis 5, wobei die Kultur die Notwendigkeit für eine antimikrobielle Verabreichung zur Behandlung der Atemwegskrankheit bei dem Kind reduziert.

7. Kultur nach einem der Ansprüche 1 bis 6, wobei die Kultur Fehlzeiten des Kinds aufgrund der Atemwegskrankheit verhindert.

8. Verwendung einer *L. acidophilus* und *B. lactis* umfassenden Kultur bei der Herstellung eines Arzneimittels zum Verringern oder Vorbeugen grippeähnlicher Symptome bei einem von einer Atemwegsinfektion betroffenen Kind, wobei es sich bei dem *L. acidophilus-Stamm* um NCFM handelt und bei dem *B.* lactis-Stamm um *B. lactis* Bi-07 handelt.

9. Verwendung nach Anspruch 8, wobei es sich bei dem *B.* lactis-Stamm um *B. lactis* Bi-07 handelt.

10. Verwendung nach einem der Ansprüche 8 und 9, wobei die Kultur dem Kind oral verabreicht wird, vorzugsweise als Nahrungsergänzungsmittel.

11. Verwendung nach einem der Ansprüche 8 bis 10, wobei die Kultur die Notwendigkeit für eine antimikrobielle Verabreichung zur Behandlung der Atemwegskrankheit bei dem Kind reduziert.

## Revendications

1. Culture comprenant *L. acidophilus* et *B. lactis* pour utilisation dans la réduction ou la prévention des symptômes grippaux chez un enfant affecté par une infection des voies respiratoires, où *L. acidophilus* est la souche NCFM et la souche *B. lactis* est *B. lactis* Bi-07.

2. Culture selon la revendication 1, où ledit enfant est âgé d'environ 3 ans à environ 5 ans.

3. Culture selon l'une quelconque des revendications 1 et 2, où ladite culture est destinée à l'administration pendant les mois d'automne ou d'hiver.

4. Culture selon l'une quelconque des revendications 1 à 3, où ladite culture est destinée à l'administration audit enfant par voie orale.

5. Culture selon l'une quelconque des revendications 1 à 4, où ladite culture se présente sous la forme d'un supplément nutritionnel.

6. Culture selon l'une quelconque des revendications 1 à 5, où ladite culture réduit la nécessité d'une administration antimicrobienne pour traiter ladite maladie respiratoire chez ledit enfant.

7. Culture selon l'une quelconque des revendications 1 à 6, où ladite culture prévient l'absentéisme dû à ladite maladie respiratoire chez ledit enfant.

8. Utilisation d'une culture comprenant *L. acidophilus* et *B. lactis* dans la fabrication d'un médicament destiné à la réduction ou la prévention des symptômes grippaux chez un enfant affecté par une infection des voies respiratoires, où *L. acidophilus* est la souche NCFM et la souche *B. lactis* est *B. lactis* Bi-07.

9. Utilisation selon la revendication 8, où ladite culture est administrée audit enfant par voie orale, préférentiellement sous forme de supplément nutritionnel.

10. Utilisation selon l'une quelconque des revendications 8 et 9, où ladite culture réduit la nécessité d'une administration antimicrobienne pour traiter ladite maladie respiratoire chez ledit enfant.

11. Utilisation selon l'une quelconque des revendications 8 à 10, où l'administration de ladite culture réduit l'absentéisme dû à ladite maladie respiratoire chez ledit enfant.
